# EUROPEAN PATENT APPLICATION

(11) **EP 4 537 824 A1**
(43) Date of publication of application: **16.04.2025**
(21) Application number: 23819909.5
(22) Date of filing: 09.06.2023
(51) Int. Cl.: A61K 31/437, A61K 9/36, A61K 47/38, A61P 35/00, A61P 43/00

(54) **PHARMACEUTICAL COMPOSITION CONTAINING PIMITESPIB**

(30) Priority: 10.06.2022 JP 2022094420
(71) Applicant: Taiho Pharmaceutical Co., Ltd., Chiyoda-ku, Tokyo 101-8444 (JP)
(72) Inventor: NISHIYAMA, Tetsuya, Tokushima-shi, Tokushima 771-0194 (JP); NIKI, Yuuichirou, Tokushima-shi, Tokushima 771-0194 (JP)
(74) Representative: Wächtershäuser & Hartz Patentanwaltspartnerschaft mbB
(86) International application number: PCT/JP2023/021535
(87) International publication number: WO 2023/238929

(57) **Abstract**

Provided is a pharmaceutical composition containing compound 1, the pharmaceutical composition having excellent disintegratability and bioavailability. The pharmaceutical composition comprises: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and crystalline cellulose, and has a disintegration time of within 360 seconds in a coated tablet form.

## Description

### Field of the Invention

The present invention relates to a pharmaceutical composition, particularly, a pharmaceutical composition for oral administration, containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide (hereinafter, also referred to as "compound 1") or a pharmaceutically acceptable salt thereof and crystalline cellulose and a method for producing the same.

### Background of the Invention

A group of proteins called molecular chaperons functions variously in such a way that the proteins promote or maintain the formation of the functional structures of other proteins, promote correct association, suppress unnecessary aggregation, protect other proteins from degradation, and promote secretion. HSP90 is a molecular chaperone as abundant as approximately 1 to 2% of all intracellular soluble proteins and is unnecessary for biosynthesis of the majority of polypeptides, unlike other chaperon proteins (Non Patent Literature 1). Signaling-related factors (e.g., ERBB1/EGFR, ERBB2/HER2, MET, IGF1R, KDR/VEGFR, FLT3, ZAP70, KIT, CHUK/IKK, BRAF, RAF1, SRC, and AKT), cell cycle regulatory factors (e.g., CDK4, CDK6, Cyclin D, PLK1, and BIRC5), and transcriptional regulators (e.g., HIF-1α, p53, androgen receptor, estrogen receptor, and progesterone receptor) are known as main client proteins whose structural formation or stability is controlled through interaction with HSP90 (Non Patent Literatures 2 and 3). HSP90 is deeply involved in cell proliferation or survival by maintaining the normal functions of these proteins. Further, HSP90 is required for the normal functions of mutated or chimeric factors (for example, BCR-ABL and NPM-ALK) which cause carcinogenesis or exacerbation of cancer. This indicates the importance of HSP90 particularly for processes such as carcinogenesis, cancer survival, growth, exacerbation, and metastasis (Non Patent Literature 2).

A plurality of HSP90 inhibitors are currently reported as antitumor agents. Patent Literature 1 discloses compound 1 which has an excellent HSP90 inhibitory effect and exhibits antitumor activity. Patent Literatures 2 to 6 disclose the application of compound 1 and application in which compound 1 is used in combination with other anticancer agents. Patent Literature 7 discloses a crystal polymorph of compound 1.

In general, pharmaceutical compositions for oral administrations are required to have not only the stability of an active ingredient but excellent disintegratability and bioavailability when orally administered.

However, an approach to solving the problem described above varies depending on the structure and properties of an active ingredient, the type of a preparation, and others. It is therefore not easy to find an optimum pharmaceutical formulation having excellent disintegratability and bioavailability.

### Citation List

### Patent Literature

Patent Literature 1: WO 2011/004610
Patent Literature 2: WO 2015/046498
Patent Literature 3: WO 2019/004417
Patent Literature 4: WO 2019/054465
Patent Literature 5: WO 2019/220512
Patent Literature 6: WO 2019/221086
Patent Literature 7: WO 2016/181990

### Non Patent Literature

Non Patent Literature 1: Nat. Rev. Cancer, 5: 761-772 (2005)
Non Patent Literature 2: TRENDS Mol. Med., 10: 283-290 (2004)
Non Patent Literature 3: Clin Can Res 15, 9-14 (2009)

### Summary of the Invention

### Problems to be Solved by the Invention

Compound 1 is a compound having an excellent HSP90 inhibitory effect and antitumor activity. Accordingly, the present invention relates to providing a pharmaceutical composition containing compound 1, the pharmaceutical composition having excellent disintegratability and bioavailability.

### Means for Solving the Problem

The present inventors have conducted various studies on the disintegratability and bioavailability of compound 1 or a pharmaceutically acceptable salt thereof by studying various methods for producing a pharmaceutical composition containing compound 1 or a pharmaceutically acceptable salt thereof, and adding various compounds to the pharmaceutical composition, and consequently revealed that in the case of using a direct tableting method, compound 1 tends to result in failure of tableting, such as sticking, presumably attributed to the characteristics of compound 1. Accordingly, as a result of conducting further studies, it has been found that a pharmaceutical composition having excellent disintegratability and bioavailability is obtained by granulating compound 1 or a pharmaceutically acceptable salt thereof, and adding predetermined crystalline cellulose to the granulated product, followed by tableting.

Specifically, the present invention relates to the following [1] to [17].
[1] A pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and crystalline cellulose, and having a disintegration time of within 360 seconds in a coated tablet form.
[2] A pharmaceutical composition obtained by mixing a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof with 20.0 to 55.0% by mass of crystalline cellulose, wherein the crystalline cellulose before mixing has an L/D ratio of from 1.00 to 4.00 and/or a bulk density of from 0.20 to 0.50 g/cm³.
[3] A pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and 20.0 to 55.0% by mass of crystalline cellulose selected from the group consisting of Ceolus PH-102, PH-302, KG-802, and UF-711.
[4] The pharmaceutical composition according to [2] or [3], wherein a content of the crystalline cellulose is from 30.0 to 55.0% by mass.
[5] The pharmaceutical composition according to any of [2] to [4], wherein the pharmaceutical composition has a disintegration time of within 180 seconds in an uncoated tablet form or a disintegration time of within 360 seconds in a coated tablet form.
[6] The pharmaceutical composition according to any of [1] to [5], wherein an additive other than the crystalline cellulose to be added outside the granulated product is only an additive selected from the group consisting of a binder, a lubricant, a fluidizer, a colorant, a flavor, a corrigent, a sweetener, a brightener, and a plasticizer.
[7] The pharmaceutical composition according to any of [1] to [5], wherein an additive other than the crystalline cellulose to be added outside the granulated product is only a lubricant.
[8] The pharmaceutical composition according to any of [1] to [7], wherein the pharmaceutical composition contains 18.0 to 40.0% by mass of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof.
[9] The pharmaceutical composition according to any of [1] to [8], wherein the granulated product is a granulated product produced by a wet granulation method.
[10] The pharmaceutical composition according to any of [1] to [9], wherein an average particle size (d50) of the granulated product is from 50 to 400 µm.
[11] The pharmaceutical composition according to any of [1] to [10], wherein the pharmaceutical composition is a solid preparation, preferably a solid preparation for oral administration.
[12] The pharmaceutical composition according to [11], wherein the pharmaceutical composition is a tablet.
[13] The pharmaceutical composition according to [12], wherein a diameter of the tablet is from 6.5 to 9.5 mm.
[14] A coated composition prepared by coating the pharmaceutical composition according to any of [1] to [13].
[15] A method for producing a pharmaceutical composition, comprising granulating a powder containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof, with a binder liquid; and mixing a granulated product obtained in the granulating step with crystalline cellulose having an L/D ratio of from 1.00 to 4.00 and/or a bulk density of from 0.20 to 0.50 g/cm³ such that a content of the crystalline cellulose is from 20.0 to 55.0% by mass in the pharmaceutical composition.
[16] The method for producing a pharmaceutical composition according to [15], wherein a method of the granulating is a fluidized-bed granulation method.
[17] A pharmaceutical composition produced by the method according to [15] or [16].

The present invention also relates to the following [18] to [54].
[18] The pharmaceutical composition according to any of [1] to [13] or the coated composition according to [14] for the treatment of tumor.
[19] Use of a pharmaceutical composition for producing a medicament for the treatment of tumor, the pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and crystalline cellulose, and having a disintegration time of within 360 seconds in a coated tablet form.
[20] Use of a pharmaceutical composition for producing a medicament for the treatment of tumor, the pharmaceutical composition being a pharmaceutical composition obtained by mixing a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof with 20.0 to 55.0% by mass of crystalline cellulose, wherein the crystalline cellulose before mixing has an L/D ratio of from 1.00 to 4.00 and/or a bulk density of from 0.20 to 0.50 g/cm³.
[21] Use of a pharmaceutical composition for producing a medicament for the treatment of tumor, the pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and 20.0 to 55.0% by mass of crystalline cellulose selected from the group consisting of Ceolus PH-102, PH-302, KG-802, and UF-711.
[22] The use according to [20] or [21], wherein a content of the crystalline cellulose in the pharmaceutical composition is from 30.0 to 55.0% by mass.
[23] The use according to any of [20] to [22], wherein the pharmaceutical composition has a disintegration time of within 180 seconds in an uncoated tablet form or a disintegration time of within 360 seconds in a coated tablet form.
[24] The use according to any of [19] to [23], wherein the pharmaceutical composition contains 18.0 to 40.0% by mass of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof.
[25] The use according to any of [19] to [24], wherein the granulated product is a granulated product produced by a wet granulation method.
[26] The use according to any of [19] to [25], wherein an average particle size (d50) of the granulated product is from 50 to 400 µm.
[27] The use according to any of [19] to [26], wherein the pharmaceutical composition is a solid preparation, preferably a solid preparation for oral administration.
[28] The use according to any of [19] to [27], wherein the pharmaceutical composition is a tablet.
[29] The use according to [28], wherein a diameter of the tablet is from 6.5 to 9.5 mm.
[30] The use according to any of [19] to [29], wherein the pharmaceutical composition is a coated composition prepared by coating the pharmaceutical composition.
[31] A pharmaceutical composition for use in the treatment of tumor, the pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and crystalline cellulose, and having a disintegration time of within 360 seconds in a coated tablet form.
[32] A pharmaceutical composition for use in the treatment of tumor, the pharmaceutical composition being a pharmaceutical composition obtained by mixing a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof with 20.0 to 55.0% by mass of crystalline cellulose, wherein the crystalline cellulose before mixing has an L/D ratio of from 1.00 to 4.00 and/or a bulk density of from 0.20 to 0.50 g/cm³.
[33] A pharmaceutical composition for use in the treatment of tumor, the pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and 20.0 to 55.0% by mass of crystalline cellulose selected from the group consisting of Ceolus PH-102, PH-302, KG-802, and UF-711.
[34] The pharmaceutical composition according to [32] or [33], wherein a content of the crystalline cellulose is from 30.0 to 55.0% by mass.
[35] The pharmaceutical composition according to any of [32] to [34], wherein the pharmaceutical composition has a disintegration time of within 180 seconds in an uncoated tablet form or a disintegration time of within 360 seconds in a coated tablet form.
[36] The pharmaceutical composition according to any of [31] to [35], wherein the pharmaceutical composition contains 18.0 to 40.0% by mass of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof.
[37] The pharmaceutical composition according to any of [31] to [36], wherein the granulated product is a granulated product produced by a wet granulation method.
[38] The pharmaceutical composition according to any of [31] to [37], wherein an average particle size (d50) of the granulated product is from 50 to 400 µm.
[39] The pharmaceutical composition according to any of [31] to [38], wherein the pharmaceutical composition is a solid preparation, preferably a solid preparation for oral administration.
[40] The pharmaceutical composition according to any of [31] to [39], wherein the pharmaceutical composition is a tablet.
[41] The pharmaceutical composition according to [40], wherein a diameter of the tablet is from 6.5 to 9.5 mm.
[42] The pharmaceutical composition according to any of [31] to [41], wherein the pharmaceutical composition is a coated composition prepared by coating the pharmaceutical composition.
[43] A method for treating tumor, comprising administering an effective amount of a pharmaceutical composition to a subject in need thereof, the pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and crystalline cellulose, and having a disintegration time of within 360 seconds in a coated tablet form.
[44] A method for treating tumor, comprising administering an effective amount of a pharmaceutical composition to a subject in need thereof, the pharmaceutical composition being a pharmaceutical composition obtained by mixing a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof with 20.0 to 55.0% by mass of crystalline cellulose, wherein the crystalline cellulose before mixing has an L/D ratio of from 1.00 to 4.00 and/or a bulk density of from 0.20 to 0.50 g/cm³.
[45] A method for treating tumor, comprising administering an effective amount of a pharmaceutical composition to a subject in need thereof, the pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and 20.0 to 55.0% by mass of crystalline cellulose selected from the group consisting of Ceolus PH-102, PH-302, KG-802, and UF-711.
[46] The method according to [44] or [45], wherein a content of the crystalline cellulose in the pharmaceutical composition is from 30.0 to 55.0% by mass.
[47] The method according to any of [44] to [46], wherein the pharmaceutical composition has a disintegration time of within 180 seconds in an uncoated tablet form or a disintegration time of within 360 seconds in a coated tablet form.
[48] The method according to any of [43] to [47], wherein the pharmaceutical composition contains 18.0 to 40.0% by mass of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof.
[49] The method according to any of [43] to [48], wherein the granulated product is a granulated product produced by a wet granulation method.
[50] The method according to any of [43] to [49], wherein an average particle size (d50) of the granulated product is from 50 to 400 µm.
[51] The method according to any of [43] to [50], wherein the pharmaceutical composition is a solid preparation, preferably a solid preparation for oral administration.
[52] The method according to any of [43] to [51], wherein the pharmaceutical composition is a tablet.
[53] The method according to [52], wherein a diameter of the tablet is from 6.5 to 9.5 mm.
[54] The method according to any of [43] to [53], wherein the pharmaceutical composition is a coated composition prepared by coating the pharmaceutical composition.

### Effects of the Invention

The present invention can provide a pharmaceutical composition which contains compound 1 or a pharmaceutically acceptable salt thereof, has excellent disintegratability and bioavailability, and is free from failure of tableting, such as sticking.

### Brief Description of Drawings

Figure 1 illustrates results of measuring a bulk density and a long diameter/short diameter ratio of each crystalline cellulose.
Figure 2 illustrates results of an absorption experiment in dogs.

### Detailed Description of the Invention

Hereinafter, the pharmaceutical composition according to the present invention and a method for producing the same will be described. However, the pharmaceutical composition of the present invention and the method for producing the same are not interpreted in a manner such that they are limited to the contents described in the embodiments and Examples below.

### [Pharmaceutical composition]

The pharmaceutical composition of the present invention is a pharmaceutical composition comprising: a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide (compound 1) or a pharmaceutically acceptable salt thereof; and predetermined crystalline cellulose.

In the present invention, 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide, also called pimitespib, is a compound having the following structure.

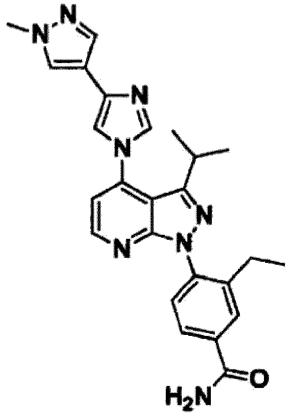

Compound 1 or the pharmaceutically acceptable salt thereof is a known compound and can be synthesized in accordance with a method described in, for example, Patent Literature 1 (WO 2011/004610).

When compound 1 has isomers such as optical isomers, stereoisomers, rotational isomers, or tautomers, any of the isomers and mixtures thereof are encompassed by compound 1 unless otherwise specified.

Compound 1 or the pharmaceutically acceptable salt thereof may be a solvate (e.g., a hydrate) or a non-solvate. In the present invention, both the solvate and the non-solvate are encompassed by "compound 1 or pharmaceutically acceptable salt thereof".

The pharmaceutically acceptable salt is a salt having desirable pharmacological activity of the compound and means a salt prepared from a pharmaceutically acceptable nontoxic base or acid including an inorganic or organic base and an inorganic or organic acid.

Examples of the pharmaceutically acceptable salt of compound 1 include, but are not particularly limited to addition salts with inorganic acids (e.g., hydrochloric acid and sulfuric acid) or organic acids (e.g., acetic acid, citric acid, tartaric acid, and maleic acid), salts with alkali metals (e.g., potassium and sodium), salts with alkaline earth metals (e.g., calcium and magnesium), and salts with organic bases such as ammonium salt, ethylamine salt, and arginine salt.

A direct tableting method is the most convenient tableting method. However, in the case of preparing compound 1 into tablets by the direct tableting method, failure of tableting occurred presumably due to the characteristics of compound 1. Granulation followed by tablet preparation were studied as a solution thereto. Since the characteristics of compounds differ among the individual compounds, it is difficult to determine a method which can be carried out for each compound, and thus it is not easy to find an optimum method for preparing tablets thereof. As shown in Examples mentioned later, tablets which solved the problem described above and had excellent disintegratability were successfully provided by granulating compound 1 or a pharmaceutically acceptable salt thereof and adding predetermined crystalline cellulose to the granulated product, followed by tableting.

In the present invention, the granulated product containing compound 1 or a pharmaceutically acceptable salt thereof is not particularly limited and may be a wet-granulated product or a dry-granulated product. The dry granulation method, which does not employ a binder, is characterized in that even drugs unstable in water can be granulated; however this method tends to increase the size of tablets. Hence, the granulated product containing compound 1 or a pharmaceutically acceptable salt thereof is preferably a wet-granulated product in view of preparing tablets as small as possible.

The granulated product containing compound 1 or a pharmaceutically acceptable salt thereof according to the present invention may contain an active ingredient other than compound 1 or the pharmaceutically acceptable salt thereof, or an additive which is generally used in preparations in the pharmaceutical field, as long as the advantageous effects of the present invention are exerted. Preferably, the granulated product contains only compound 1 or a pharmaceutically acceptable salt thereof as an active ingredient. The content of the additive can be arbitrary as long as the object of the present invention is interfered.

The additive in the granulated product may include, for example, an excipient, a binder, a disintegrant, a fluidizer, and a solubilizer.

Examples of the excipient in the granulated product include sugar alcohols such as D-mannitol, erythritol, D-sorbitol, and xylitol, trehalose hydrate, β-cyclodextrin, corn starch, sucrose, lactose, crystalline cellulose, anhydrous calcium hydrogen phosphate, and precipitated calcium carbonate. The excipient is preferably lactose, D-mannitol, crystalline cellulose, corn starch, or a combination thereof, more preferably lactose, corn starch, or a combination thereof.

Examples of the binder in the granulated product include hydroxypropylcellulose, hypromellose, povidone, and polyvinyl alcohol. Among them, the binder is preferably hydroxypropylcellulose, povidone, or a combination thereof, more preferably hydroxypropylcellulose.

The form of the granulated product containing compound 1 or a pharmaceutically acceptable salt thereof according to the present invention is preferably a granulated product containing compound 1 or a pharmaceutically acceptable salt thereof and one or more additives selected from the group consisting of lactose, D-mannitol, crystalline cellulose, corn starch, hydroxypropylcellulose, and povidone, more preferably a granulated product containing compound 1 or a pharmaceutically acceptable salt thereof and one or more additives selected from the group consisting of lactose, corn starch, and hydroxypropylcellulose, particularly preferably a granulated product containing compound 1 or a pharmaceutically acceptable salt thereof, lactose, corn starch, and hydroxypropylcellulose.

Another form of the granulated product containing compound 1 or a pharmaceutically acceptable salt thereof according to the present invention is preferably a granulated product containing only compound 1 or a pharmaceutically acceptable salt thereof as an active ingredient, and containing one or more additives selected from the group consisting of lactose, D-mannitol, crystalline cellulose, corn starch, hydroxypropylcellulose, and povidone, more preferably a granulated product composed of compound 1 or a pharmaceutically acceptable salt thereof and one or more additives selected from the group consisting of lactose, D-mannitol, crystalline cellulose, corn starch, hydroxypropylcellulose, and povidone, more preferably a granulated product composed of compound 1 or a pharmaceutically acceptable salt thereof and one or more additives selected from the group consisting of lactose, corn starch, and hydroxypropylcellulose, particularly preferably a granulated product composed of compound 1 or a pharmaceutically acceptable salt thereof, lactose, corn starch, and hydroxypropylcellulose.

In the pharmaceutical composition of the present invention, the content of compound 1 or the pharmaceutically acceptable salt thereof is preferably from 18.0 to 40.0% by mass, more preferably from 20.0 to 32.0% by mass, more preferably from 20.0 to 29.0% by mass, further more preferably from 26.0 to 29.0% by mass, particularly preferably from 26.0 to 27.0% by mass, most preferably 26.7% by mass, per 100.0% by mass in total of the pharmaceutical composition, in view of, for example, elution properties, stability, absorption properties, and ease of handling in large-scale production.

The average particle size of the granulated product containing compound 1 or a pharmaceutically acceptable salt thereof according to the present invention is not particularly limited as long as the advantageous effects of the present invention are exerted. Too small an average particle size influences the properties of tablets and increases the possibility of causing failure of tableting such as sticking. Too large an average particle size increases the possibility of causing uneven contents in tablets. The average particle size is preferably a median size (particle size at which a cumulative frequency is 50%: d₅₀) of from 50 to 400 µm, more preferably from 50 to 300 µm, more preferably from 50 to 250 µm, more preferably from 50 to 200 µm, more preferably from 60 to 180 µm, more preferably from 60 to 150 µm, particularly preferably from 80 to 120 µm. The median size of the granulated product can be measured by a known measurement approach and can be measured by, for example, a sieving method or a laser diffraction particle size distribution measurement method (wet or dry).

As described in Examples mentioned later, lactose, crystalline cellulose, and D-mannitol, which are excipients generally used in preparations in the pharmaceutical field, were comparatively studied as excipients to be added outside the granulated product containing compound 1 or a pharmaceutically acceptable salt thereof. As a result, it was found that predetermined crystalline cellulose was preferred in view of tablet hardness, moldability, and disintegratability. In particular, the disintegratability was excellent. Thus, in the pharmaceutical composition of the present invention, the excipient to be added outside the granulated product includes crystalline cellulose.

In the present invention, the crystalline cellulose to be added outside the granulated product is preferably crystalline cellulose having a bulk density of from 0.20 to 0.50 g/cm³. The bulk density of the crystalline cellulose is more preferably from 0.20 to 0.44 g/cm³, particularly preferably from 0.25 to 0.44 g/cm³, in view of disintegratability.

A commercially available product can be used as the crystalline cellulose having a bulk density of from 0.20 to 0.50 g/cm³. Specifically, Ceolus(R) PH-102, PH-302, KG-802, or UF-711 (all manufactured by Asahi Kasei Corp.) can be used, for example. The type of the crystalline cellulose is preferably Ceolus PH-102, PH-302, KG-802, or UF-711, more preferably Ceolus PH-102 or PH-302, particularly preferably Ceolus PH-102.

The "bulk density" is the density of a powder obtained by fully filling a container having a given capacity with the powder and regarding the inner capacity as a volume, and can be measured by Determination of Bulk and Tapped Densities stipulated in the Japanese Pharmacopoeia. The measurement of the bulk density may vary to a certain extent depending on the measurement conditions such as humidity during measurement. Thus, the numeric value should not be strictly interpreted. Accordingly, in the present specification, the numeric value of the bulk density may have a measurement error in a range on the order of ±0.08 g/cm³.

In the present invention, the "L/D ratio" is the ratio between the long diameter and short diameter of crystalline cellulose. The L/D ratio can be determined, for example, by measuring long diameters and short diameters of 50 particles under an electron microscope (VHX-D500, manufactured by Keyence Corp.), and calculating the ratio between average values thereof. In the present specification, the numeric value of the L/D ratio may have a measurement error in a range on the order of ±5%.

In the present invention, the crystalline cellulose to be added outside the granulated product is preferably crystalline cellulose having an L/D ratio of from 1.00 to 4.00. The L/D ratio of the crystalline cellulose is more preferably from 1.00 to 3.50, more preferably from 1.50 to 3.50, more preferably from 1.62 to 3.50, particularly preferably from 1.62 to 3.15, in view of disintegratability.

A commercially available product can be used as the crystalline cellulose having an L/D ratio of from 1.00 to 4.00. Specifically, Ceolus(R) PH-102, PH-302, KG-802, or UF-711 (all manufactured by Asahi Kasei Corp.) can be used, for example. The type of the crystalline cellulose is preferably Ceolus PH-102, PH-302, KG-802, or UF-711, more preferably Ceolus PH-102 or PH-302, particularly preferably Ceolus PH-102.

In the pharmaceutical composition of the present invention, the content of the crystalline cellulose to be added outside the granulated product is preferably from 20.0 to 55.0% by mass, more preferably from 30.0 to 55.0% by mass, more preferably from 35.0 to 55.0% by mass, further more preferably from 35.0 to 36.0% by mass, particularly preferably 35.2% by mass, per 100.0% by mass in total of the pharmaceutical composition in view of disintegratability.

The pharmaceutical composition of the present invention may contain an additive which is generally used in preparations in the pharmaceutical field, other than the crystalline cellulose, as long as the advantageous effects of the present invention are exerted.

The additive to be added outside the granulated product, other than the crystalline cellulose is not particularly limited as long as the additive is generally used in preparations in the pharmaceutical field. Examples thereof include excipients other than the crystalline cellulose, binders, disintegrants, lubricants, fluidizers, colorants, flavors, corrigents, sweeteners, brighteners, and plasticizers.

In this context, examples of the excipient and the binder include the excipients and the binders mentioned above.

Examples of the disintegrant include low substituted hydroxypropylcellulose, carmellose, corn starch, carmellose sodium, croscarmellose sodium, carmellose calcium, pregelatinized starch, and crospovidone. Among them, the disintegrant is preferably croscarmellose sodium, carmellose calcium, pregelatinized starch, crospovidone, or a combination thereof, particularly preferably croscarmellose sodium.

Examples of the lubricant include hydrogenated oil, sucrose fatty acid ester, sodium lauryl sulfate, magnesium stearate, sodium stearyl fumarate, and stearic acid. Among them, the lubricant is preferably magnesium stearate, stearic acid, or a combination thereof, particularly preferably magnesium stearate.

Examples of the colorant include Food Yellow No. 5 dyes, Food Blue No. 2 dyes, food lake dyes, iron sesquioxide, yellow ferric oxide, and titanium oxide. Among them, the colorant is preferably titanium oxide.

Examples of the fluidizer include light anhydrous silicic acid, hydrated silicon dioxide, talc, and magnesium stearate.

In the pharmaceutical composition of the present invention, a preferable mode of the additive other than the crystalline cellulose to be added outside the granulated product is preferably the absence of the excipient other than the crystalline cellulose (i.e., the excipient to be added outside the granulated product is only the crystalline cellulose). Specifically, the additive preferably includes only an additive selected from the group consisting of a binder, a disintegrant, a lubricant, a fluidizer, a colorant, a flavor, a corrigent, a sweetener, a brightener, and a plasticizer. More preferably, the additive includes no excipient other than the crystalline cellulose or a disintegrant. Specifically, the additive more preferably includes only an additive selected from the group consisting of a binder, a lubricant, a fluidizer, a colorant, a flavor, a corrigent, a sweetener, a brightener, and a plasticizer, particularly preferably includes only a lubricant.

The pharmaceutical composition of the present invention may assume various dosage forms and is preferably in a solid preparation form, particularly preferably a solid preparation for oral administration. Examples of the solid preparation include tablets (including uncoated tablets, orally disintegrating tablets, chewable tablets, and the like), capsules (including soft capsules, hard capsules, and the like), granules, powders, and pills. The solid preparation is preferably a tablet, particularly preferably an uncoated tablet.

The pharmaceutical composition of the present invention is expected to be applied to an antitumor agent and is a highly active substance. The pharmaceutical composition is preferably a coated composition prepared by coating the surface of the pharmaceutical composition of the present invention, in view of measures against hazard and compliance. In this context, the coating is not particularly limited as long as the coating can prevent exposure of an active ingredient of an uncoated tablet in the surface thereof. The coating includes, for example, film coating and sugar coating. Film coating is preferred. The uncoated tablet is a tablet obtained by tableting without coating the surface thereof. The coated tablet is a tablet obtained by coating the surface of the uncoated tablet.

Examples of the coating base include hypromellose, ethylcellulose, hydroxypropylcellulose, polyvinyl alcohol, polyethylene glycol, and saccharose. Among them, the coating base is preferably hypromellose, polyethylene glycol, or a combination thereof. Further, a coating additive may be used for the coating. Examples of the coating additive include shading agents, fluidizers, colorants, flavors, and plasticizers.

Compound 1 has an excellent HSP90 inhibitory effect and antitumor activity. Therefore, the pharmaceutical composition of the present invention is useful as a pharmaceutical composition for the prevention or treatment of tumor. The tumor is tumor on which compound 1 or the pharmaceutically acceptable salt thereof exerts an antitumor effect, more preferably malignant tumor involving HSP90. Specific examples thereof include head and neck cancer, digestive system cancer (e.g., esophageal cancer, stomach cancer, duodenal cancer, liver cancer, biliary tract cancer (e.g., gallbladder/bile duct cancer), pancreatic cancer, small intestine cancer, large intestine cancer (e.g., colorectal cancer, colon cancer, and rectal cancer), and gastrointestinal stromal tumor), lung cancer (e.g., non-small cell lung cancer and small cell lung cancer), breast cancer, ovary cancer, uterine cancer (e.g., uterine cervical cancer and endometrial cancer), kidney cancer, bladder cancer, prostate cancer, and skin cancer.

The size of the pharmaceutical composition for oral administration is a major issue for elderly people who has a reduced swallowing function, and the provision of a small easy-to-swallow preparation can contribute to improvement in the compliance of elderly people. On the other hand, for achieving excellent disintegratability or tablet hardness, it is necessary to sufficiently add, for example, an excipient, a binder, a disintegrant, and a lubricant depending on the characteristics of an active ingredient. Thus, it is difficult to reduce the size of tablets while achieving excellent disintegratability or tablet hardness. In general, a diameter of 10 mm requires attention to be paid to a swallowing function of subjects, and a diameter of 6 mm is difficult to handle for elderly people due to the small size (Japanese Journal of Geriatrics, Vol. 44, No. 5, 627-633 (2007: 9)).

Thus, the diameter of the pharmaceutical composition of the present invention is preferably from 6.5 to 9.5 mm, more preferably from 7.0 to 9.0 mm, particularly preferably from 7.0 to 8.0 mm, in view of easy swallowing and easy grasp.

In the present invention, the "diameter" is a diameter in a general sense for round tablets, and a long diameter for oblong (e.g., oval) tablets. The diameter of a tablet can be measured by a usually known measurement method.

The disintegration time of the pharmaceutical composition of the present invention is preferably within 180 seconds, more preferably within 120 seconds, for uncoated tablets. The disintegration time is preferably within 360 seconds, more preferably within 300 seconds, for coated tablets. As described in Examples mentioned later, such an excellent disintegration time can be achieved by blending a specific amount of specific crystalline cellulose outside the granulated product according to the present invention.

The "disintegration time" can be measured by Disintegration Test stipulated in the Japanese Pharmacopoeia. The disintegration time can also be measured by Disintegration Test for tablets or for coated tablets stipulated in the Japanese Pharmacopoeia depending on a dosage form to be measured. More specifically, the disintegration time can be measured using a disintegration tester under conditions involving testing solution: water (37 ± 2°C) and no disc, the disintegration time being defined as the time from placement of a tablet in the tester to complete disintegration of the tablet.

A preferable mode of the disintegration time of the pharmaceutical composition of the present invention is preferably within 180 seconds, more preferably within 120 seconds, in an uncoated tablet form as measured by Disintegration Test stipulated in the Japanese Pharmacopeia, or is preferably within 360 seconds, more preferably within 300 seconds, in a coated tablet form as measured by Disintegration Test stipulated in the Japanese Pharmacopeia.

Another preferable mode of the disintegration time of the pharmaceutical composition of the present invention is preferably within 180 seconds, more preferably within 120 seconds, in an uncoated tablet form as measured using a disintegration tester under conditions involving testing solution: water (37 ± 2°C) and no disc, the disintegration time being defined as the time from placement of a tablet in the tester to complete disintegration of the tablet, or is preferably within 360 seconds, more preferably within 300 seconds, in a coated tablet form as measured using a disintegration tester under conditions involving testing solution: water (37 ± 2°C) and no disc, the disintegration time being defined as the time from placement of a tablet in the tester to complete disintegration of the tablet.

The hardness of the pharmaceutical composition of the present invention is 50 N or more, preferably 60 N or more, more preferably 70 N or more, further more preferably 80 N or more, in view of moldability and possible failure at the time of transport. The "hardness" is a specification known in the pharmaceutical field and can be measured, for example, for tablets, by using a tablet hardness scale (e.g., PC-30, manufactured by Okada Seiko Co., Ltd., and 8M, Schleuniger), gradually pressurizing a tablet from the side with the equipment, and determining a load (N) at which the tablet is broken.

The pharmaceutical composition of the present invention has excellent disintegratability and therefore exerts drug efficacy by exerting sufficient bioavailability in the body of a patient. In this context, the "bioavailability" is an index for an amount in which an administered drug is systemically circulated, and means the rate of compound 1 systemically circulating from an orally administered pharmaceutical composition containing compound 1. The bioavailability can be determined from, for example, absorption properties in pharmacokinetics in dogs as shown in Examples mentioned later.

A preferable mode of the pharmaceutical composition of the present invention is a pharmaceutical composition comprising: a granulated product containing compound 1 or a pharmaceutically acceptable salt thereof; and crystalline cellulose, and having a disintegration time of within 360 seconds in a coated tablet form,
more preferably a pharmaceutical composition: comprising a granulated product containing only compound 1 or a pharmaceutically acceptable salt thereof as an active ingredient; and crystalline cellulose; and including no excipient other than the crystalline cellulose as an additive other than the crystalline cellulose to be added outside the granulated product, and having a disintegration time of within 360 seconds in a coated tablet form,
more preferably a pharmaceutical composition: comprising a granulated product containing only compound 1 or a pharmaceutically acceptable salt thereof as an active ingredient, and containing one or more additives selected from the group consisting of lactose, D-mannitol, crystalline cellulose, corn starch, hydroxypropylcellulose, and povidone; and crystalline cellulose; and including no excipient other than the crystalline cellulose as an additive other than the crystalline cellulose to be added outside the granulated product, and having a disintegration time of within 360 seconds in a coated tablet form,
particularly preferably a pharmaceutical composition consisting of: a granulated product composed of compound 1 or a pharmaceutically acceptable salt thereof, lactose, corn starch, and hydroxypropylcellulose; crystalline cellulose; and a lubricant, the pharmaceutical composition having a disintegration time of within 360 seconds in a coated tablet form.

A preferable mode of the pharmaceutical composition of the present invention is a pharmaceutical composition comprising: a granulated product containing compound 1 or a pharmaceutically acceptable salt thereof; and crystalline cellulose, and having a disintegration time of within 360 seconds in a coated tablet form as measured by Disintegration Test stipulated in the Japanese Pharmacopeia,
more preferably a pharmaceutical composition: comprising a granulated product containing only compound 1 or a pharmaceutically acceptable salt thereof as an active ingredient; and crystalline cellulose; and including no excipient other than the crystalline cellulose as an additive other than the crystalline cellulose to be added outside the granulated product, and having a disintegration time of within 360 seconds in a coated tablet form as measured by Disintegration Test stipulated in the Japanese Pharmacopeia,
more preferably a pharmaceutical composition: comprising a granulated product containing only compound 1 or a pharmaceutically acceptable salt thereof as an active ingredient, and containing one or more additives selected from the group consisting of lactose, D-mannitol, crystalline cellulose, corn starch, hydroxypropylcellulose, and povidone; and crystalline cellulose; and including no excipient other than the crystalline cellulose as an additive other than the crystalline cellulose to be added outside the granulated product, and having a disintegration time of within 360 seconds in a coated tablet form as measured by Disintegration Test stipulated in the Japanese Pharmacopeia,
particularly preferably a pharmaceutical composition consisting of: a granulated product composed of compound 1 or a pharmaceutically acceptable salt thereof, lactose, corn starch, and hydroxypropylcellulose; crystalline cellulose; and a lubricant, the pharmaceutical composition having a disintegration time of within 360 seconds in a coated tablet form as measured by Disintegration Test stipulated in the Japanese Pharmacopeia.

A preferable mode of the pharmaceutical composition of the present invention is a pharmaceutical composition comprising: a granulated product containing compound 1 or a pharmaceutically acceptable salt thereof; and crystalline cellulose, and having a disintegration time of within 360 seconds in a coated tablet form as measured using a disintegration tester under conditions involving testing solution: water (37 ± 2°C) and no disc, the disintegration time being defined as the time from placement of the tablet in the tester to complete disintegration of the tablet,
more preferably a pharmaceutical composition: comprising a granulated product containing only compound 1 or a pharmaceutically acceptable salt thereof as an active ingredient; and crystalline cellulose; and including no excipient other than the crystalline cellulose as an additive other than the crystalline cellulose to be added outside the granulated product, and having a disintegration time of within 360 seconds in a coated tablet form as measured using a disintegration tester under conditions involving testing solution: water (37 ± 2°C) and no disc, the disintegration time being defined as the time from placement of the tablet in the tester to complete disintegration of the tablet,
more preferably a pharmaceutical composition: comprising a granulated product containing only compound 1 or a pharmaceutically acceptable salt thereof as an active ingredient, and containing one or more additives selected from the group consisting of lactose, D-mannitol, crystalline cellulose, corn starch, hydroxypropylcellulose, and povidone; and crystalline cellulose; and including no excipient other than the crystalline cellulose as an additive other than the crystalline cellulose to be added outside the granulated product, and having a disintegration time of within 360 seconds in a coated tablet form as measured using a disintegration tester under conditions involving testing solution: water (37 ± 2°C) and no disc, the disintegration time being defined as the time from placement of the tablet in the tester to complete disintegration of the tablet,
particularly preferably a pharmaceutical composition consisting of: a granulated product composed of compound 1 or a pharmaceutically acceptable salt thereof, lactose, corn starch, and hydroxypropylcellulose; crystalline cellulose; and a lubricant, the pharmaceutical composition having a disintegration time of within 360 seconds in a coated tablet form as measured using a disintegration tester under conditions involving testing solution: water (37 ± 2°C) and no disc, the disintegration time being defined as the time from placement of the tablet in the tester to complete disintegration of the tablet.

Another preferable mode of the pharmaceutical composition of the present invention is a pharmaceutical composition obtained by mixing a granulated product containing compound 1 or a pharmaceutically acceptable salt thereof with 20.0 to 55.0% by mass of crystalline cellulose, wherein the crystalline cellulose before mixing has an L/D ratio of 1.00 to 4.00 and/or a bulk density of 0.20 to 0.50 g/cm³,
more preferably a pharmaceutical composition obtained by mixing a granulated product containing only compound 1 or a pharmaceutically acceptable salt thereof as an active ingredient with 20.0 to 55.0% by mass of crystalline cellulose, wherein the crystalline cellulose before mixing has an L/D ratio of 1.00 to 4.00 and/or a bulk density of 0.20 to 0.50 g/cm³, wherein the crystalline cellulose before mixing has an L/D ratio of 1.00 to 4.00 and/or a bulk density of 0.20 to 0.50 g/cm³, and the pharmaceutical composition includes no excipient other than the crystalline cellulose as an additive other than the crystalline cellulose to be added outside the granulated product,
more preferably a pharmaceutical composition obtained by mixing a granulated product containing only compound 1 or a pharmaceutically acceptable salt thereof as an active ingredient, and containing one or more additives selected from the group consisting of lactose, D-mannitol, crystalline cellulose, corn starch, hydroxypropylcellulose, and povidone with 20.0 to 55.0% by mass of crystalline cellulose, wherein the crystalline cellulose before mixing has an L/D ratio of 1.00 to 4.00 and/or a bulk density of 0.20 to 0.50 g/cm³, and the pharmaceutical composition includes no excipient other than the crystalline cellulose as an additive other than the crystalline cellulose to be added outside the granulated product,
particularly preferably a pharmaceutical composition obtained by mixing a granulated product composed of compound 1 or a pharmaceutically acceptable salt thereof, lactose, corn starch, and hydroxypropylcellulose with only 20.0 to 55.0% by mass of crystalline cellulose and a lubricant, wherein the crystalline cellulose before mixing has an L/D ratio of 1.00 to 4.00 and/or a bulk density of 0.20 to 0.50 g/cm³.

Still another preferable mode of the pharmaceutical composition of the present invention is a pharmaceutical composition comprising: a granulated product containing compound 1 or a pharmaceutically acceptable salt thereof; and 20.0 to 55.0% by mass of one or more crystalline celluloses selected from the group consisting of Ceolus PH-102, PH-302, KG-802, and UF-711,
more preferably a pharmaceutical composition comprising: a granulated product containing only compound 1 or a pharmaceutically acceptable salt thereof as an active ingredient; and 20.0 to 55.0% by mass of one or more crystalline celluloses selected from the group consisting of Ceolus PH-102, PH-302, KG-802, and UF-711, the pharmaceutical composition including no excipient other than the crystalline cellulose as an additive other than the crystalline cellulose to be added outside the granulated product,
more preferably a pharmaceutical composition comprising: a granulated product containing only compound 1 or a pharmaceutically acceptable salt thereof as an active ingredient, and containing one or more additives selected from the group consisting of lactose, D-mannitol, crystalline cellulose, corn starch, hydroxypropylcellulose, and povidone; and 20.0 to 55.0% by mass of one or more crystalline celluloses selected from the group consisting of Ceolus PH-102, PH-302, KG-802, and UF-711, the pharmaceutical composition including no excipient other than the crystalline cellulose as an additive other than the crystalline cellulose to be added outside the granulated product,
particularly preferably a pharmaceutical composition consisting of: a granulated product composed of compound 1 or a pharmaceutically acceptable salt thereof, lactose, corn starch, and hydroxypropylcellulose; 20.0 to 55.0% by mass of one or more crystalline celluloses selected from the group consisting of Ceolus PH-102, PH-302, KG-802, and UF-711; and a lubricant.

### [Method for producing pharmaceutical composition]

The pharmaceutical composition of the present invention can be preferably obtained by a production method comprising granulating a powder containing compound 1 or a pharmaceutically acceptable salt thereof with a binder liquid; and mixing a granulated product obtained in the granulating step with crystalline cellulose such that a content of the crystalline cellulose is from 20.0 to 55.0% by mass in the pharmaceutical composition.

The powder containing compound 1 or a pharmaceutically acceptable salt thereof may contain an active ingredient other than compound 1 or the pharmaceutically acceptable salt thereof or the aforementioned additive which is generally used in preparations in the pharmaceutical field.

In the present invention, examples of the granulation method include wet granulation methods such as fluidized-bed granulation methods, stirring granulation methods, tumbling fluidized granulation methods, extrusion granulation methods, spray granulation methods, and milling granulation methods. Among them, a fluidized-bed granulation method is preferred.

The binder liquid used for granulation is preferably a solution obtained by dissolving or dispersing the aforementioned binder in water.

After granulation, the obtained granulated product may be dried, or the particle size of the granulated product may be adjusted with a particle size selector or the like.

Subsequently, the obtained granulated product is mixed with crystalline cellulose. The crystalline cellulose is as mentioned above.

The mixing ratio between the granulated product and the crystalline cellulose is amounts such that the content of the crystalline cellulose in the pharmaceutical composition is from 20.0 to 55.0% by mass, preferably from 30.0 to 55.0% by mass, more preferably from 35.0 to 55.0% by mass, further more preferably from 35.0 to 36.0% by mass, particularly preferably 35.2% by mass, per 100.0% by mass in total of the pharmaceutical composition.

The mixing ratio is amounts such that the content of compound 1 or the pharmaceutically acceptable salt thereof in the pharmaceutical composition is preferably from 18.0 to 40.0% by mass, more preferably from 20.0 to 32.0% by mass, further more preferably from 20.0 to 29.0% by mass, further more preferably from 26.0 to 29.0% by mass, particularly preferably from 26.0 to 27.0% by mass, most preferably 26.7% by mass, per 100.0% by mass in total of the pharmaceutical composition.

In this way, a pharmaceutical composition comprising: a granulated product containing compound 1 or a pharmaceutically acceptable salt thereof; and 20.0 to 55.0% by mass of crystalline cellulose can be obtained.

The pharmaceutical composition may be administered directly as granules, a powder, or the like. In the case of formulating the pharmaceutical composition as tablets, the tablets can be produced, for example, by mixing the granulated product with crystalline cellulose, followed by tableting. The tableting is performed by using a known tableting machine, and appropriately adjusting a tableting pressure and others such that the resulting tablets have moderate hardness and can disintegrate rapidly as the pharmaceutical composition. For example, a rotary tableting machine, a single-punch tableting machine, or an oil hydraulic press can be appropriately selected and used as the tableting machine.

The pharmaceutical composition may be coated, if necessary. The coating base and the coating additive are as mentioned above.

All publications, patents, and patent applications cited herein are incorporated herein by reference in their entirety.

Next, the present invention will be described in detail by way of Examples. However, the present invention is not limited by these examples.

### Examples

### [Reference Example 1: production of granulated product]

A fluidized-bed granulator "FLC-3N-5" (manufactured by Freund Corp.) was charged with 2 000 g of pimitespib (manufactured by Alps Pharmaceutical Ind. Co., Ltd.), 1 925 g of lactose hydrate (Lactochem fine powder, manufactured by DMV Fonterra Excipients GmbH & Co. KG), and 750 g of corn starch (Nisshoku Corn Starch W, manufactured by Nihon Shokuhin Kako Co., Ltd.), and fluidized-bed granulation was performed with a 1 500 g aliquot of a binder liquid obtained by dissolving 300 g of hydroxypropylcellulose (HPC SSL, manufactured by Nippon Soda Co., Ltd.) in 2 700 g of purified water. The particle size was regulated using a sieve (opening: 600 µm) to obtain a granulated product.

### [Reference Example 2: production of tablet i]

A fluidized-bed granulator "FL-LABO" (manufactured by Freund Corp.) was charged with 100 g of pimitespib (manufactured by Alps Pharmaceutical Ind. Co., Ltd.), 96.25 g of lactose hydrate (Lactochem fine powder, manufactured by DMV Fonterra Excipients GmbH & Co. KG), and 37.5 g of corn starch (Nisshoku Corn Starch W, manufactured by Nihon Shokuhin Kako Co., Ltd.), and fluidized-bed granulation was performed with a 75 g aliquot of a binder liquid obtained by dissolving 30 g of hydroxypropylcellulose (HPC SSL, manufactured by Nippon Soda Co., Ltd.) in 270 g of purified water. The particle size was regulated using a sieve (opening: 600 µm) to obtain a granulated product. A 193 g aliquot of the obtained granulated product was supplemented and thoroughly mixed with 6 g of croscarmellose sodium (Ac-Di-Sol SD-711, manufactured by FMC Health and Nutrition) and 1 g of magnesium stearate (specially manufactured, vegetable, manufactured by Taihei Chemical Industrial Co., Ltd.), and the resulting mixture was tableted at a compression force of 5 kN using a rotary tableting machine "VELAG" (manufactured by Kikusui Seisakusho Ltd.) to obtain a tablet having a diameter of 6 mm and a mass of 100 mg.

### [Reference Example 3: production of tablet ii]

A 1.93 g aliquot of the granulated product obtained in Reference Example 1 was supplemented and thoroughly mixed with 1 g of lactose hydrate (Supertab 11SD, manufactured by DMV Fonterra Excipients GmbH & Co. KG), 0.06 g of croscarmellose sodium (Ac-Di-Sol SD-711, manufactured by FMC Health and Nutrition), and 0.01 g of magnesium stearate (specially manufactured, vegetable, manufactured by Taihei Chemical Industrial Co., Ltd.), and the resulting mixture was tableted at a compression force of 0.75 ton using an oil hydraulic press (manufactured by Riken Seiki Co., Ltd.) to obtain a tablet having a diameter of 7 mm and a mass of 150 mg.

### [Reference Example 4: production of tablet iii]

A 1.93 g aliquot of the granulated product obtained in Reference Example 1 was supplemented and thoroughly mixed with 1 g of crystalline cellulose (Ceolus KG-802, manufactured by Asahi Kasei Corp.), 0.06 g of croscarmellose sodium (Ac-Di-Sol SD-711, manufactured by FMC Health and Nutrition), and 0.01 g of magnesium stearate (specially manufactured, vegetable, manufactured by Taihei Chemical Industrial Co., Ltd.), and the resulting mixture was tableted at a compression force of 2 kN using a precision universal tester Autograph (manufactured by Shimadzu Corp.) to obtain a tablet having a diameter of 7 mm and a mass of 150 mg.

### [Reference Example 5: production of tablet iv]

A 1.93 g aliquot of the granulated product obtained in Reference Example 1 was supplemented and thoroughly mixed with 1 g of D-mannitol (Pearlitol 100SD, Roquette), 0.06 g of croscarmellose sodium (Ac-Di-Sol SD-711, manufactured by FMC Health and Nutrition), and 0.01 g of magnesium stearate (specially manufactured, vegetable, manufactured by Taihei Chemical Industrial Co., Ltd.), and the resulting mixture was tableted at a compression force of 3.5 kN using a precision universal tester Autograph (manufactured by Shimadzu Corp.) to obtain a tablet having a diameter of 7 mm and a mass of 150 mg.

### [Test Example 1: measurement of hardness and disintegration time]

The hardness in the diameter direction was measured on the tablet produced in Reference Example 2 using a tablet hardness scale (8M, Schleuniger), and the hardness (N) was determined from an average value from five tablets. The disintegration time was measured using a disintegration tester (NT-4H(P), manufactured by Toyama Sangyo Co., Ltd.) under conditions involving testing solution: water (37 ± 2°C) and no disc, the disintegration time being defined as the time from placement of the tablet in the tester to complete disintegration of the tablet.

The hardness in the diameter direction was measured on the tablet produced in Reference Example 3 using a tablet hardness scale (8M, Schleuniger), and the hardness (N) was determined from an average value from three tablets. The disintegration time was measured using a disintegration tester (NT-4H, manufactured by Toyama Sangyo Co., Ltd.) under conditions involving testing solution: water (37 ± 2°C) and no disc, the disintegration time being defined as the time from placement of the tablet in the tester to complete disintegration of the tablet.

The hardness in the diameter direction was measured on the tablets produced in Reference Examples 4 and 5 using a tablet hardness scale (PC-30, manufactured by Okada Seiko Co., Ltd.), and the hardness (N) was determined from an average value from three tablets. The disintegration time was measured using a disintegration tester (NT-4H, manufactured by Toyama Sangyo Co., Ltd.) under conditions involving testing solution: water (37 ± 2°C) and no disc, the disintegration time being defined as the time from placement of the tablet in the tester to complete disintegration of the tablet.

The results are shown in Table 1.

**[Table 1]**

| | Reference Example 2 Tablet i | Reference Example 3 Tablet ii | Reference Example 4 Tablet iii | Reference Example 5 Tablet iv |
|---|---|---|---|---|
| Granulated product | 96.5 | 96.5 | 96.5 | 96.5 |
| Croscarmellose sodium | 3 | 3 | 3 | 3 |
| Lactose hydrate | | 50 | | |
| Crystalline cellulose | | | 50 | |
| D-Mannitol | | | | 50 |
| Magnesium stearate | 0.5 | 0.5 | 0.5 | 0.5 |
| Total (mg) | 100 | 150 | 150 | 150 |
| Hardness (N) | 57 | 88 | 67 | 78 |
| Disintegration time (sec) | 262 | 107 | 40 | 65 |

As seen from Table 1, the tablet of Reference Example 2 disintegrated gradually from its periphery when disintegration behavior was observed, suggesting that no water was introduced inside the tablet. On the other hand, it was confirmed that the tablets of Reference Example 3, Reference Example 4, and Reference Example 5 disintegrated into several fragments in the disintegration tester, and it was thus demonstrated that the disintegration time and the disintegration behavior were improved by adding a sufficient amount of an excipient to between granules. The formulation was optimized on the basis of Reference Example 4, which was found to have a more improved disintegration time.

### [Example 1: production of tablet A]

A 96.5 g aliquot of the granulated product obtained in Reference Example 1 was supplemented and thoroughly mixed with 52.75 g of crystalline cellulose (Ceolus PH-102, manufactured by Asahi Kasei Corp.) and 0.75 g of magnesium stearate (specially manufactured, vegetable, manufactured by Taihei Chemical Industrial Co., Ltd.), and the resulting mixture was tableted at a tableting pressure of 4 kN using a rotary tableting machine "VELAG" (manufactured by Kikusui Seisakusho Ltd.) so as to attain a mass of 150 mg, 40 mg of pimitespib, and 52.75 mg of crystalline cellulose (Ceolus PH-102) per tablet to obtain a tablet.

A coating machine "HC-FZ-LABO" (manufactured by Freund Corp.) was charged with the tableted product, which was then coated with a coating solution obtained by dissolving 28.8 g of hypromellose (TC-5R, manufactured by Shin-Etsu Chemical Co., Ltd.), 3.6 g of macrogol 6 000 (macrogol 6 000P, manufactured by NOF Corp.), and 3.6 g of titanium oxide (Titanium Oxide NA63, manufactured by Toho Titanium Co., Ltd.) in 324 g of purified water so as to attain a mass of 150 mg, 3.6 mg of hypromellose, 0.45 mg of macrogol 6 000, and 0.45 mg of titanium oxide per tablet, to obtain a coated tablet having a diameter of 7.5 mm and a mass of 154.5 mg.

### [Example 2: production of tablet B]

A 96.5 g aliquot of the granulated product obtained in Reference Example 1 was supplemented and thoroughly mixed with 52.75 g of crystalline cellulose (Ceolus PH-302, manufactured by Asahi Kasei Corp.) and 0.75 g of magnesium stearate (specially manufactured, vegetable, manufactured by Taihei Chemical Industrial Co., Ltd.), and the resulting mixture was tableted at a tableting pressure of 5 kN using a rotary tableting machine "VELAG" (manufactured by Kikusui Seisakusho Ltd.) so as to attain a mass of 150 mg, 40 mg of pimitespib, and 52.75 mg of crystalline cellulose (Ceolus PH-302) per tablet to obtain a tablet.

A coating machine "HC-FZ-LABO" (manufactured by Freund Corp.) was charged with the tableted product, which was then coated with a coating solution obtained by dissolving 64.8 g of hypromellose (TC-5R, manufactured by Shin-Etsu Chemical Co., Ltd.), 8.1 g of macrogol 6 000 (macrogol 6 000P, manufactured by NOF Corp.), and 8.1 g of titanium oxide (Titanium Oxide NA63, manufactured by Toho Titanium Co., Ltd.) in 729 g of purified water so as to attain a mass of 150 mg, 3.6 mg of hypromellose, 0.45 mg of macrogol 6 000, and 0.45 mg of titanium oxide per tablet, to obtain a coated tablet having a diameter of 7.5 mm and a mass of 154.5 mg.

### [Example 3: production of tablet C]

A 96.5 g aliquot of the granulated product obtained in Reference Example 1 was supplemented and thoroughly mixed with 52.75 g of crystalline cellulose (Ceolus KG-802, manufactured by Asahi Kasei Corp.) and 0.75 g of magnesium stearate (specially manufactured, vegetable, manufactured by Taihei Chemical Industrial Co., Ltd.), and the resulting mixture was tableted at a tableting pressure of 3 kN using a rotary tableting machine "VELAG" (manufactured by Kikusui Seisakusho Ltd.) so as to attain a mass of 150 mg, 40 mg of pimitespib, and 52.75 mg of crystalline cellulose (Ceolus KG-802) per tablet to obtain a tablet.

A coating machine "HC-FZ-LABO" (manufactured by Freund Corp.) was charged with the tableted product, which was then coated with a coating solution obtained by dissolving 28.8 g of hypromellose (TC-5R, manufactured by Shin-Etsu Chemical Co., Ltd.), 3.6 g of macrogol 6 000 (macrogol 6 000P, manufactured by NOF Corp.), and 3.6 g of titanium oxide (Titanium Oxide NA63, manufactured by Toho Titanium Co., Ltd.) in 324 g of purified water so as to attain a mass of 150 mg, 3.6 mg of hypromellose, 0.45 mg of macrogol 6 000, and 0.45 mg of titanium oxide per tablet, to obtain a coated tablet having a diameter of 7.5 mm and a mass of 154.5 mg.

### [Example 4: production of tablet D]

A 96.5 g aliquot of the granulated product obtained in Reference Example 1 was supplemented and thoroughly mixed with 52.75 g of crystalline cellulose (Ceolus UF-711, manufactured by Asahi Kasei Corp.) and 0.75 g of magnesium stearate (specially manufactured, vegetable, manufactured by Taihei Chemical Industrial Co., Ltd.), and the resulting mixture was tableted at a tableting pressure of 3.5 kN using a rotary tableting machine "VELAG" (manufactured by Kikusui Seisakusho Ltd.) so as to attain a mass of 150 mg, 40 mg of pimitespib, and 52.75 mg of crystalline cellulose (Ceolus UF-711) per tablet to obtain a tablet.

A coating machine "HC-FZ-LABO" (manufactured by Freund Corp.) was charged with the tableted product, which was then coated with a coating solution obtained by dissolving 28.8 g of hypromellose (TC-5R, manufactured by Shin-Etsu Chemical Co., Ltd.), 3.6 g of macrogol 6 000 (macrogol 6 000P, manufactured by NOF Corp.), and 3.6 g of titanium oxide (Titanium Oxide NA63, manufactured by Toho Titanium Co., Ltd.) in 324 g of purified water so as to attain a mass of 150 mg, 3.6 mg of hypromellose, 0.45 mg of macrogol 6 000, and 0.45 mg of titanium oxide per tablet, to obtain a coated tablet having a diameter of 7.5 mm and a mass of 154.5 mg.

### [Comparative Example 1: production of tablet a]

A 96.5 g aliquot of the granulated product obtained in Reference Example 1 was supplemented and thoroughly mixed with 52.75 g of crystalline cellulose (Ceolus KG-1 000, manufactured by Asahi Kasei Corp.) and 0.75 g of magnesium stearate (specially manufactured, vegetable, manufactured by Taihei Chemical Industrial Co., Ltd.), and the resulting mixture was tableted at a tableting pressure of 3 kN using a rotary tableting machine "VELAG" (manufactured by Kikusui Seisakusho Ltd.) so as to attain a mass of 150 mg, 40 mg of pimitespib, and 52.75 mg of crystalline cellulose (Ceolus KG-1 000) per tablet to obtain a tablet.

A coating machine "HC-FZ-LABO" (manufactured by Freund Corp.) was charged with the tableted product, which was then coated with a coating solution obtained by dissolving 28.8 g of hypromellose (TC-5R, manufactured by Shin-Etsu Chemical Co., Ltd.), 3.6 g of macrogol 6 000 (macrogol 6 000P, manufactured by NOF Corp.), and 3.6 g of titanium oxide (Titanium Oxide NA63, manufactured by Toho Titanium Co., Ltd.) in 324 g of purified water so as to attain a mass of 150 mg, 3.6 mg of hypromellose, 0.45 mg of macrogol 6 000, and 0.45 mg of titanium oxide per tablet, to obtain a coated tablet having a diameter of 7.5 mm and a mass of 154.5 mg.

### [Comparative Example 2: production of tablet b]

A 125.45 g aliquot of the granulated product obtained in Reference Example 1 was supplemented and thoroughly mixed with 0.62 g of magnesium stearate (specially manufactured, vegetable, manufactured by Taihei Chemical Industrial Co., Ltd.), and the resulting mixture was tableted at a tableting pressure of 4 kN using a rotary tableting machine "VELAG" (manufactured by Kikusui Seisakusho Ltd.) so as to attain a mass of 96.98 mg and 40 mg of pimitespib per tablet to obtain a tablet.

A coating machine "HC-FZ-LABO" (manufactured by Freund Corp.) was charged with the tableted product, which was then coated with a coating solution obtained by dissolving 23.3 g of hypromellose (TC-5R, manufactured by Shin-Etsu Chemical Co., Ltd.), 2.9 g of macrogol 6 000 (macrogol 6 000P, manufactured by NOF Corp.), and 2.9 g of titanium oxide (Titanium Oxide NA63, manufactured by Toho Titanium Co., Ltd.) in 261.8 g of purified water so as to attain a mass of 96.98 mg, 2.33 mg of hypromellose, 0.29 mg of macrogol 6 000, and 0.29 mg of titanium oxide per tablet, to obtain a coated tablet having a diameter of 6 mm and a mass of 99.89 mg.

### [Example 5: production of tablet E]

A 96.5 g aliquot of the granulated product obtained in Reference Example 1 was supplemented and thoroughly mixed with 27.88 g of crystalline cellulose (Ceolus PH-102, manufactured by Asahi Kasei Corp.) and 0.63 g of magnesium stearate (specially manufactured, vegetable, manufactured by Taihei Chemical Industrial Co., Ltd.), and the resulting mixture was tableted at a tableting pressure of 4 kN using a rotary tableting machine "VELAG" (manufactured by Kikusui Seisakusho Ltd.) so as to attain a mass of 125 mg, 40 mg of pimitespib, and 27.875 mg of crystalline cellulose (Ceolus PH-102) per tablet to obtain a tablet.

A coating machine "HC-FZ-LABO" (manufactured by Freund Corp.) was charged with the tableted product, which was then coated with a coating solution obtained by dissolving 64.8 g of hypromellose (TC-5R, manufactured by Shin-Etsu Chemical Co., Ltd.), 8.1 g of macrogol 6 000 (macrogol 6 000P, manufactured by NOF Corp.), and 8.1 g of titanium oxide (Titanium Oxide NA63, manufactured by Toho Titanium Co., Ltd.) in 729 g of purified water so as to attain a mass of 125 mg, 3 mg of hypromellose, 0.375 mg of macrogol 6 000, and 0.375 mg of titanium oxide per tablet, to obtain a coated tablet having a diameter of 7 mm and a mass of 128.75 mg.

### [Example 6: production of tablet F]

A 96.5 g aliquot of the granulated product obtained in Reference Example 1 was supplemented and thoroughly mixed with 42.8 g of crystalline cellulose (Ceolus PH-102, manufactured by Asahi Kasei Corp.) and 0.7 g of magnesium stearate (specially manufactured, vegetable, manufactured by Taihei Chemical Industrial Co., Ltd.), and the resulting mixture was tableted at a tableting pressure of 4.5 kN using a rotary tableting machine "VELAG" (manufactured by Kikusui Seisakusho Ltd.) so as to attain a mass of 140 mg, 40 mg of pimitespib, and 42.8 mg of crystalline cellulose (Ceolus PH-102) per tablet to obtain a tablet.

A coating machine "HC-FZ-LABO" (manufactured by Freund Corp.) was charged with the tableted product, which was then coated with a coating solution obtained by dissolving 64.8 g of hypromellose (TC-5R, manufactured by Shin-Etsu Chemical Co., Ltd.), 8.1 g of macrogol 6 000 (macrogol 6 000P, manufactured by NOF Corp.), and 8.1 g of titanium oxide (Titanium Oxide NA63, manufactured by Toho Titanium Co., Ltd.) in 729 g of purified water so as to attain a mass of 140 mg, 3.36 mg of hypromellose, 0.42 mg of macrogol 6 000, and 0.42 mg of titanium oxide per tablet, to obtain a coated tablet having a diameter of 7.5 mm and a mass of 144.2 mg.

### [Example 7: production of tablet G]

A 96.5 g aliquot of the granulated product obtained in Reference Example 1 was supplemented and thoroughly mixed with 102.5 g of crystalline cellulose (Ceolus PH-102, manufactured by Asahi Kasei Corp.) and 1 g of magnesium stearate (specially manufactured, vegetable, manufactured by Taihei Chemical Industrial Co., Ltd.), and the resulting mixture was tableted at a tableting pressure of 3.5 kN using a rotary tableting machine "VELAG" (manufactured by Kikusui Seisakusho Ltd.) so as to attain a mass of 200 mg, 40 mg of pimitespib, and 102.5 mg of crystalline cellulose (Ceolus PH-102) per tablet to obtain a tablet.

A coating machine "HC-FZ-LABO" (manufactured by Freund Corp.) was charged with the tableted product, which was then coated with a coating solution obtained by dissolving 64.8 g of hypromellose (TC-5R, manufactured by Shin-Etsu Chemical Co., Ltd.), 8.1 g of macrogol 6 000 (macrogol 6 000P, manufactured by NOF Corp.), and 8.1 g of titanium oxide (Titanium Oxide NA63, manufactured by Toho Titanium Co., Ltd.) in 729 g of purified water so as to attain a mass of 200 mg, 4.8 mg of hypromellose, 0.6 mg of macrogol 6 000, and 0.6 mg of titanium oxide per tablet, to obtain a coated tablet having a diameter of 8 mm and a mass of 206 mg.

### [Comparative Example 3: production of tablet c]

A 96.5 g aliquot of the granulated product obtained in Reference Example 1 was supplemented and thoroughly mixed with 301.5 g of crystalline cellulose (Ceolus PH-102, manufactured by Asahi Kasei Corp.) and 2 g of magnesium stearate (specially manufactured, vegetable, manufactured by Taihei Chemical Industrial Co., Ltd.), and the resulting mixture was tableted at a tableting pressure of 3.5 kN using a rotary tableting machine "VELAG" (manufactured by Kikusui Seisakusho Ltd.) so as to attain a mass of 400 mg, 40 mg of pimitespib, and 301.5 mg of crystalline cellulose (Ceolus PH-102) per tablet to obtain a tablet.

A coating machine "HC-FZ-LABO" (manufactured by Freund Corp.) was charged with the tableted product, which was then coated with a coating solution obtained by dissolving 64.8 g of hypromellose (TC-5R, manufactured by Shin-Etsu Chemical Co., Ltd.), 8.1 g of macrogol 6 000 (macrogol 6 000P, manufactured by NOF Corp.), and 8.1 g of titanium oxide (Titanium Oxide NA63, manufactured by Toho Titanium Co., Ltd.) in 729 g of purified water so as to attain a mass of 400 mg, 9.6 mg of hypromellose, 1.2 mg of macrogol 6 000, and 1.2 mg of titanium oxide per tablet, to obtain a coated tablet having a diameter of 10 mm and a mass of 412 mg.

### [Test Example 2: measurement of disintegration time]

The disintegration time was measured on the tablets produced in Examples 1 to 7 and Comparative Examples 1 to 3 using a disintegration tester (NT-4H or NT-4H(P), manufactured by Toyama Sangyo Co., Ltd.) under conditions involving testing solution: water (37 ± 2°C) and no disc, the disintegration time being defined as the time from placement of the tablet in the tester to complete disintegration of the tablet.

The results are shown in Tables 2 and 3.

**[Table 2]**

| | Example 1 Tablet A | Example 2 Tablet B | Example 3 Tablet C | Example 4 Tablet D | Comparative Example 1 Tablet a |
|---|---|---|---|---|---|
| Granulated product | 96.5 | 96.5 | 96.5 | 96.5 | 96.5 |
| Ceolus PH-102 | 52.75 | | | | |
| Ceolus PH-302 | | 52.75 | | | |
| Ceolus KG-802 | | | 52.75 | | |
| Ceolus UF-711 | | | | 52.75 | |
| Ceolus KG-1000 | | | | | 52.75 |
| Magnesium stearate | 0.75 | 0.75 | 0.75 | 0.75 | 0.75 |
| Hypromellose | 3.6 | 3.6 | 3.6 | 3.6 | 3.6 |
| Macrogol 6000P | 0.45 | 0.45 | 0.45 | 0.45 | 0.45 |
| Titanium oxide | 0.45 | 0.45 | 0.45 | 0.45 | 0.45 |
| Total (mg) | 154.5 | 154.5 | 154.5 | 154.5 | 154.5 |
| Disintegration time (sec) (uncoated tablet) | 50 | 65 | 122 | 94 | 656 |
| Disintegration time (sec) (coated tablet) | 176 | 154 | 268 | 230 | 978 |

**[Table 3]**

| | Example 1 Tablet A | Comparative Example 2 Tablet b | Example 5 Tablet E | Example 6 Tablet F | Example 7 Tablet G | Comparative Example 3 Tablet c |
|---|---|---|---|---|---|---|
| Granulated product | 96.5 | 96.5 | 96.5 | 96.5 | 96.5 | 96.5 |
| Ceolus PH-102 | 52.75 | - | 27.875 | 42.8 | 102.5 | 301.5 |
| Magnesium stearate | 0.75 | 0.48 | 0.625 | 0.7 | 1.0 | 2.0 |
| Hypromellose | 3.6 | 2.33 | 3 | 3.36 | 4.8 | 9.6 |
| Macrogol 6000P | 0.45 | 0.29 | 0.375 | 0.42 | 0.6 | 1.2 |
| Titanium oxide | 0.45 | 0.29 | 0.375 | 0.42 | 0.6 | 1.2 |
| Total (mg) | 154.5 | 99.89 | 128.75 | 144.2 | 206 | 412 |
| Disintegration time (sec) (uncoated tablet) | 50 | 310 | 160 | 89 | 45 | 90 |
| Disintegration time (sec) (coated tablet) | 176 | 407 | 283 | 199 | 113 | 373 |
| Diameter (mm) | 7.5 | 6.0 | 7.0 | 7.5 | 8.0 | 10.0 |

The coated tablets obtained by coating the uncoated tablets of Table 2 (Examples 1 to 4 and Comparative Example 1) were evaluated for the disintegration times of the crystalline celluloses differing in physical properties. As a result, it was confirmed that the coated tablets of Examples 1 and 2 had a disintegration time of 3 minutes or shorter, and that the coated tablets of Examples 3 and 4 had a disintegration time of 5 minutes or shorter. On the other hand, the coated tablet of Comparative Example 1 was found to require 15 minutes or longer as a disintegration time. The coated tablets of Examples 1 and 2 had the most improved disintegration time. However, when the tableting pressure at the time of tableting was confirmed, the tableting pressure of Example 1 was approximately 4 kN and the tableting pressure of Example 2 was 5 kN. Therefore, the amount of the crystalline cellulose added was evaluated on the basis of Example 1 in view of moldability.

The uncoated tablets of Table 3 (Examples 1 and 5 to 7 and Comparative Examples 2 and 3) were evaluated for their disintegration times. As a result, in Examples 1 and 5 to 7 and Comparative Example 2, the disintegration time was found to be shorter according to the amount of the crystalline cellulose added. A large amount of the crystalline cellulose added, as in Comparative Example 3, was found to delay the disintegration time. The disintegration times of the uncoated tablets and the disintegration times of the coated tablets both had the same tendency. The amount of the crystalline cellulose added was considered to be desirably from 27.875 to 102.5 mg. For improvement in disintegratability, the addition of a disintegrant is usually studied as the first choice, but surprisingly, a composition having excellent disintegratability was successfully obtained by merely adding crystalline cellulose and adjusting the amount of the crystalline cellulose added, without further adding a disintegrant.

The sizes of the coated tablets were compared among Examples 1 and 5 to 7 and Comparative Examples 2 and 3. As a result, the coated tablet of Comparative Example 3 was 10 mm, whereas all the coated tablets of Examples 1 and 5 to 7 were as small as 8 mm or smaller. Tablets, particularly, of 7 to 8 mm, are small and easy to swallow and reportedly have good compliance. The coated tablets of Examples 1 and 5 to 7 are 7 to 8 mm and can thus be expected to serve as tablets having good compliance.

Failure of tableting, such as sticking, was not found in the tablets of Examples of the present invention.

### [Test Example 3: measurement of bulk density and long diameter/short diameter ratio]

The bulk density of each crystalline cellulose (Ceolus PH-102, PH-302, KG-802, UF-711, and KG-1 000, manufactured by Asahi Kasei Corp.) was determined in accordance with Determination of Bulk and Tapped Densities stipulated in the Japanese Pharmacopoeia using a powder tester (PT-R, manufactured by Hosokawa Micron Corp.). The long diameter/short diameter ratio was determined by measuring the long diameters and short diameters of 50 particles under an electron microscope (VHX-D500, manufactured by Keyence Corp.).

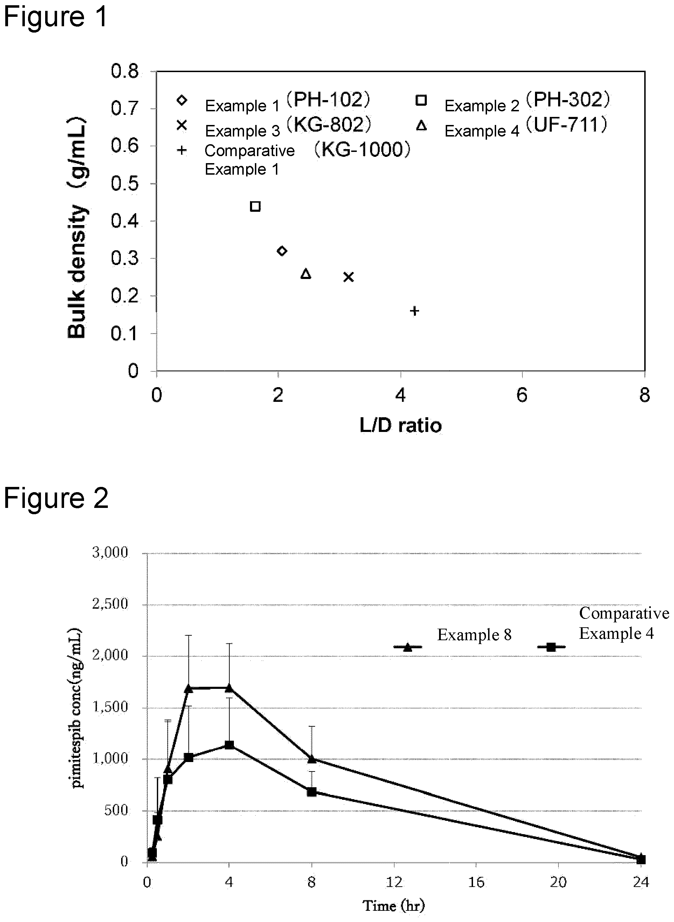

The results are shown in Figure 1.

### [Example 8: production of tablet H]

A fluidized-bed granulator "FLC-3N-5" (manufactured by Freund Corp.) was charged with 3 000 g of pimitespib (manufactured by Alps Pharmaceutical Ind. Co., Ltd.), 2 887.5 g of lactose hydrate (Lactochem fine powder, manufactured by DMV Fonterra Excipients GmbH & Co. KG), and 1 125 g of corn starch (Nisshoku Corn Starch W, manufactured by Nihon Shokuhin Kako Co., Ltd.), and fluidized-bed granulation was performed with a 2 250 g aliquot of a binder liquid obtained by dissolving 400 g of hydroxypropylcellulose (HPC SSL, manufactured by Nippon Soda Co., Ltd.) in 3 600 g of purified water. The particle size was regulated using a particle size selector "QC-U10" (manufactured by Powrex Corp.) at an impeller speed of 400 min⁻¹ to obtain a granulated product.

A 144.75 g aliquot of the obtained granulated product was supplemented and thoroughly mixed with 79.125 g of crystalline cellulose (Ceolus PH-102, manufactured by Asahi Kasei Corp.) and 1.125 g of magnesium stearate (specially manufactured, vegetable, manufactured by Taihei Chemical Industrial Co., Ltd.), and the resulting mixture was tableted using a rotary tableting machine "VELAG" (manufactured by Kikusui Seisakusho Ltd.) so as to attain a mass of 150 mg, 40 mg of pimitespib, 52.75 mg of crystalline cellulose (Ceolus PH-102), and hardness of 70 N per tablet.

A coating machine "HC-FZ-LABO" (manufactured by Freund Corp.) was charged with the tableted product, which was then coated with a coating solution obtained by dissolving 40 g of hypromellose (TC-5R, manufactured by Shin-Etsu Chemical Co., Ltd.), 5 g of macrogol 6 000 (macrogol 6 000P, manufactured by NOF Corp.), and 5 g of titanium oxide (Titanium Oxide NA63, manufactured by Toho Titanium Co., Ltd.) in 450 g of purified water so as to attain a mass of 154.5 mg, 3.6 mg of hypromellose, 0.45 mg of macrogol 6 000, and 0.45 mg of titanium oxide per tablet, to obtain a coated tablet having a diameter of 7 mm and a mass of 154.5 mg.

### [Comparative Example 4: production of tablet d]

A fluidized-bed granulator "FLC-3N-5" (manufactured by Freund Corp.) was charged with 3 000 g of pimitespib (manufactured by Alps Pharmaceutical Ind. Co., Ltd.), 2 887.5 g of lactose hydrate (Lactochem fine powder, manufactured by DMV Fonterra Excipients GmbH & Co. KG), and 1 125 g of corn starch (Nisshoku Pharmacopoeia Corn Starch, manufactured by Nihon Shokuhin Kako Co., Ltd.), and fluidized-bed granulation was performed with a 2 250 g aliquot of a binder liquid obtained by dissolving 450 g of hydroxypropylcellulose (HPC SSL, manufactured by Nippon Soda Co., Ltd.) in 4 050 g of purified water. The particle size was regulated using a particle size selector "QC-U10" (manufactured by Powrex Corp.) at an impeller speed of 400 min⁻¹to obtain a granulated product.

A 5 795.15 g aliquot of the granulated product was supplemented and thoroughly mixed with 180.16 g of croscarmellose sodium (Ac-Di-Sol SD-711, manufactured by FMC Health and Nutrition) and 30.03 g of magnesium stearate (specially manufactured, vegetable, manufactured by Taihei Chemical Industrial Co., Ltd.), and the resulting mixture was tableted using a rotary tableting machine "AQUARIUS C-H" (manufactured by Kikusui Seisakusho Ltd.) so as to attain a mass of 100 mg, 40 mg of pimitespib, 3 mg of croscarmellose sodium, and hardness of 60 N per tablet.

A coating machine "PRC-10GTX" (manufactured by Powrex Corp.) was charged with the tableted product, which was then coated with a coating solution obtained by dissolving 360 g of hypromellose (TC-5R, manufactured by Shin-Etsu Chemical Co., Ltd.), 45 g of macrogol 6 000 (macrogol 6 000P, manufactured by NOF Corp.), and 45 g of titanium oxide (Titanium Oxide NA63, manufactured by Toho Titanium Co., Ltd.) in 4 050 g of purified water so as to attain a mass of 103 mg, 2.4 mg of hypromellose, 0.3 mg of macrogol 6 000, and 0.3 mg of titanium oxide per tablet, to obtain a coated tablet having a diameter of 6 mm and a mass of 103 mg.

Table 4 shows the composition of the coated tablets of Example 8 and Comparative Example 4.

**[Table 4]**

| | Example 8 Tablet H | Comparative Example 4 Tablet d |
|---|---|---|
| Pimitespib | 40 | 40 |
| Lactose hydrate | 38.5 | 38.5 |
| Corn starch | 15 | 15 |
| Hydroxypropylcellulose | 3 | 3 |
| Crystalline cellulose | 52.75 | - |
| Croscarmellose sodium | - | 3 |
| Magnesium stearate | 0.75 | 0.5 |
| Hypromellose | 3.6 | 2.4 |
| Macrogol 6000P | 0.45 | 0.3 |
| Titanium oxide | 0.45 | 0.3 |
| Magnesium stearate | 0.0045 | 0.003 |
| Total (mg) | 154.5045 | 103.003 |

### [Test Example 4: absorption experiment in dog]

The coated tablets produced in Example 8 and Comparative Example 4 were each administered at a dose of two tablets together with 50 mL of water per individual (80 mg/body) to beagles (Kitayama Labes Co., Ltd., six males, approximately 9 to 11 kg, 39 to 44 months old; fasted; intravenously given an intravenous injection of atropine sulfate (20 µg/0.04 mL/kg) and intramuscularly given of an intramuscular injection of pentagastrin (10 µg/0.1 mL/kg), 30 minutes before medication, and then additionally given at the same dose as above of the intramuscular injection of pentagastrin twice at a 45-minute interval). For blood collection, approximately 1 mL of blood was collected from the forearm cutaneous vein using a heparin-treated syringe and needle (21 G) 0.25, 0.5, 1, 2, 4, 8, and 24 hours after administration. The collected blood was centrifuged (12 000 rpm, 5°C, 3 min), and 50 µL of the obtained plasma was collected into a polypropylene microtube, and supplemented with 50 µL of acetonitrile and 100 µL of an internal standard solution, followed by mixing in a vortex mixer. After centrifugation (20 400 g, 5°C, 1 min), 100 µL of the supernatant was collected into a polypropylene autosampler tube, and supplemented with 200 µL of water, followed by mixing in a vortex mixer. The obtained sample was quantified using a high-performance liquid chromatograph/mass spectrometer (AB Sciex Pte. Ltd.), and a Tmax value, a Cmax value, and AUC were calculated.

### <Liquid chromatography measurement conditions>

Apparatus: high-performance liquid chromatograph/mass spectrometer
(AB Sciex Pte. Ltd.)
Column: Imtakt Unison uk-c18 (2.0 mm i.d. × 50 mm, 3 µm)
Column temperature: 40°C
Mobile phase A: 10 mmol/L ammonium formate solution
Mobile phase B: acetonitrile
Sending of solution for mobile phase: the mixing ratio between the mobile phase A and the mobile phase B was adjusted to 67:33 or 65:35, and the solution was then sent from a pump. Flow rate: 0.3 mL/min
MS/MS measurement conditions
Ionization source: electrospray ionization (esi)-positive
Scan mode: multiple reaction monitoring (MRM)

The coated tablets of Table 4 (Example 8 and Comparative Example 4) were subjected to an absorption experiment using beagles (six males, approximately 9 to 11 kg, 39 to 44 months old). As a result, the relative BA of Example 8 to Comparative Example 4 was 146%, demonstrating that absorption properties are improved by using crystalline cellulose instead of a disintegrant (Figure 2 and

Table 5).

**[Table 5]**

| | Tmax (hr) | Cmax (ng/mL) | AUC_{0-24 hr} (ng/hr/mL) |
|---|---|---|---|
| Example 8 Tablet H | 2.7 ±1.0 | 1820.0 ±344.6 | 18774.4 ±4574.6 |
| Comparative Example 4 Tablet d | 2.8 ±1.3 | 1283.3 ±507.6 | 12837.4 ±3528.9 |

## Claims

1. A pharmaceutical composition comprising:
- a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and
- crystalline cellulose, and having a disintegration time of within 360 seconds in a coated tablet form.

2. A pharmaceutical composition obtained by mixing a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof with 20.0 to 55.0% by mass of crystalline cellulose, wherein the crystalline cellulose before mixing has an L/D ratio of from 1.00 to 4.00 and/or a bulk density of from 0.20 to 0.50 g/cm³.

3. A pharmaceutical composition comprising:
- a granulated product containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof; and
- 20.0 to 55.0% by mass of crystalline cellulose selected from the group consisting of Ceolus PH-102, PH-302, KG-802, and UF-711.

4. The pharmaceutical composition according to claim 2 or 3,
wherein a content of the crystalline cellulose is from 30.0 to 55.0% by mass.

5. The pharmaceutical composition according to any one of claims 2 to 4, wherein the pharmaceutical composition has a disintegration time of within 180 seconds in an uncoated tablet form or a disintegration time of within 360 seconds in a coated tablet form.

6. The pharmaceutical composition according to any one of claims 1 to 5, wherein the pharmaceutical composition contains 18.0 to 40.0% by mass of 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof.

7. The pharmaceutical composition according to any one of claims 1 to 6, wherein the granulated product is a granulated product produced by a wet granulation method.

8. The pharmaceutical composition according to any one of claims 1 to 7, wherein an average particle size (d50) of the granulated product is from 50 to 400 µm.

9. The pharmaceutical composition according to any one of claims 1 to 8, wherein the pharmaceutical composition is a solid preparation.

10. The pharmaceutical composition according to claim 9, wherein the pharmaceutical composition is a tablet.

11. The pharmaceutical composition according to claim 10, wherein a diameter of the tablet is from 6.5 to 9.5 mm.

12. A coated composition prepared by coating the pharmaceutical composition according to any one of claims 1 to 11.

13. A method for producing a pharmaceutical composition, comprising:
- granulating a powder containing 3-ethyl-4-{4-[4-(1-methyl-1H-pyrazol-4-yl)-1H-imidazol-1-yl]-3-(propan-2-yl)-1H-pyrazolo[3,4-b]pyridin-1-yl}benzamide or a pharmaceutically acceptable salt thereof, with a binder liquid; and
- mixing a granulated product obtained in the granulating step with crystalline cellulose having an L/D ratio of from 1.00 to 4.00 and/or a bulk density of from 0.20 to 0.50 g/cm³ such that a content of the crystalline cellulose is from 20.0 to 55.0% by mass in the pharmaceutical composition.

14. The method for producing a pharmaceutical composition according to claim 13, wherein a method of the granulating is a fluidized-bed granulation method.

15. A pharmaceutical composition produced by the method according to claim 13 or 14.
